# EUROPÄISCHE PATENTSCHRIFT

(11) **EP 1 526 101 B1**
(45) Veröffentlichungstag und Bekanntmachung des Hinweises auf die Patenterteilung: **07.03.2007**
(21) Anmeldenummer: 05001514.8
(22) Anmeldetag: 29.01.1999
(51) Int. Cl.: B65H 35/06, B65H 39/16, A61K 9/00

(54) **Verfahren zum Einbringen einer Vielzahl vereinzelter folienförmiger Darreichungsformen in einen Spender unter Bildung eines viellagigen Stapels**
Method for inserting a plurality of individual sheetlike dosage forms in a dispenser by forming a multilayer pile
Procédé pour l'introduction d'une pluralité de formes posologiques individuelles dans un distributeur, en formant une pile multicouche

(30) Priorität: 19.02.1998 DE 19806966
(43) Veröffentlichungstag der Anmeldung: 27.04.2005
(62) Teilanmeldung aus: 03014212.9
(73) Patentinhaber: LTS LOHMANN Therapie-Systeme AG, 56626 Andernach (DE)
(72) Erfinder: Steinborn, Peter, 56567 Neuwied (DE); Dzekan, Horst, 56584 Meinborn (DE); Schumann, Klaus, 56567 Neuwied (DE); Laux, Wolfgang, 65582 Diez (DE); Horstmann, Michael, 56564 Neuwied (DE)
(74) Vertreter: Flaccus, Rolf-Dieter

(56) Entgegenhaltungen:
- DE-A1- 1 461 280
- GB-A- 2 223 001
- US-A- 2 788 208
- US-A- 3 823 934
- US-A- 4 738 847

## Beschreibung

Die Erfindung betrifft ein Verfahren zum Einbringen einer Vielzahl vereinzelter folienförmiger Darreichungsformen in einen Spender unter Bildung eines viellagigen Stapels zur einzeldosierbaren Entnahme, wobei als Ausgangsmaterial in Rollen aufgewickelte folienförmige Bänder vorliegen. Die Darreichungsformen können für ihre Anwendung therapeutische oder kosmetische bzw. lebensmitteltechnische Produkte enthalten.

Im Mundbereich und auf den Schleimhäuten des Mundes anzuwendende flächige Darreichungsformen sind bekannt. So beschreibt die US 3 444 858 (1969) Medikamentstreifen auf Basis eines gelatineartigen Materials.

Weiterhin sind Anwendungsvorschläge solcher Folien außerhalb des Arzneimittelbereiches bekannt. In der EP 0 216 762 wird eine wasserlösliche Folie aus Stärke, Gelatine, Glycerin oder Sorbit offenbart, die mittels eines Walzenauftrags beschichtet wird. Dabei wird erwähnt, daß sich solche Dosierformen z.B. auch für chemische Reagenzien, Aromastoffe und dergleichen herstellen lassen.

Auf dem Markt haben sich seit ca. 1995 erhältliche Darreichungsformen im kosmetischen und Süßwaren-Anwendungsbereich mit Einzelspendern aus Kunststoff eingebürgert; erwähnt sei das Produkt der Nisshin, Japan. Die Einzelspender enthalten Stapel aufeinanderliegender Folienabschnitte, die nach Öffnung einer Öffnungslippe die Einzelentnahme des jeweils obersten Folienstückes erlauben.

Die technische Lösung der Erzeugung und Verpackung solcher Stapel aus folienförmigem Ausgangsmaterial stößt jedoch auf Schwierigkeiten; der Literatur sind keine praktikablen Anleitungen zu ihrer Herstellung zu entnehmen. Lediglich US 4 180 558 beschreibt die Stapelung eßbarer Folien, die aber an den Kanten aufeinanderlaminiert sind und sich daher für die vorstehende Anwendungsweise nicht eignen. Weil solche folienförmigen Darreichungsformen wegen ihres geringen Flächengewichts, beispielsweise zwischen 10 und 50 g/m², zu statischer Aufladung neigen und die Oberflächen zwecks leichter Entnahme verschiebbar ausgeführt sein müssen, gelingt ein exakt positioniertes Schneiden und Aufeinanderlegen aus Folienabschnitten nur mit großen Schwierigkeiten und mit großem Zeitaufwand.

Auch die Beobachtung von Herstelltechniken industrieller Bereiche wie der Papierverarbeitung oder der Kunststoffbeutelverpackung liefert keine praktikablen Hinweise zur Problemlösung. In der US 4 070 014 wird zwar die Bildung von Stapeln aus zwei verschiedenen Papiersorten in teilweise überlappender Anordnung mit Hilfe vakuumansaugender Transferrollen beschrieben. Diese Technik, wie auch die aus der US 5 348 527 bekannte Technik der Papierstaplung beschreibt ein vergleichsweise zeitaufwendiges Verfahren von Einzelschnitten aus einer Rolle und Einzelablage auf einen Stapel.
Die US 4 406 650 beschreibt die Bildung eines in Zick-Zack-Form gefalteten Papierbandes von einer Rolle, aus der ein Stapel durch Beschnitt hervorgeht. Hier liegt ein Nachteil im Verschnitt des Materials. Bei unterschiedlicher Folienstruktur der Ober- und Unterseite oder bei Wellung gelingt es dabei nicht, die Einzelblätter mit ihrer ursprünglichen Oberseite nach oben zu stapeln.

Ausgehend vom vorgenannten Stand der Technik liegt der Erfindung die Aufgabe zugrunde, ein Verfahren der im Oberbegriff von Anspruch 1 genannten Art anzugeben, mit dem es unter Vermeidung der vorgenannten Nachteile und Schwierigkeiten gelingt, einzelne dosierbare folienförmige Darreichungsformen präzise und mit hoher Arbeitsgeschwindigkeit in einen Spender unter Bildung eines viellagigen Stapels einzubringen, wobei als Ausgangsmaterial in Rollen aufgewickelte folienförmige Bänder vorliegen.

Diese Aufgabe wird mit der Erfindung bei einem Verfahren der im Oberbegriff von Anspruch 1 genannten Art durch die im Kennzeichnungsteil angegebene Folge von Arbeitsschritten gelöst.
Dabei wird mit Vorteil in überraschend einfacher Arbeitsweise der Stapel durch gleichzeitiges Abwickeln mehrerer Einzelrollen des folienförmigen Zwischenproduktes, Aufeinanderlegen der Einzelbahnen und gemeinsames Querschneiden des entstandenen Mehrfachlaminatstranges und Ablängen der Stapel exakt gebildet und in den Spender eingeschoben. Mit großem Vorteil eignet sich das erfindungsgemäße Verfahren für ein genaues Arbeiten mit großer Geschwindigkeit.

Das hierfür vorgesehene Zwischenprodukt der Schmalrollen besteht beispielsweise aus ca. 20 bis 1000 m langen, aufgerollten Einzelbahnen in Produktbreite, welche durch Rollenschnitt aus ursprünglicher Breitware herstellbar sind.
Im einfachsten Fall wird hierfür eine Anzahl von Schmalrollen, welche der Anzahl von Folienstücken pro Spender entspricht, in der Nähe eines herzustellenden Stapels frei rollbar auf Achsen fixiert.

Die Dreh- und Abwickelrichtung der Rollen ist bevorzugt gleichsinnig, und das Material wird mittels dem Fachmann bekannter Zugeinrichtungen von allen Rollen gemeinsam abgezogen und geführt. Der entstandene Mehrfachlaminatstrang wird unter Kontrolle und Egalisierung der seitlichen Stoßkanten sodann einer Querschnitteinrichtung zugeführt, hinter welcher sich der zu beschickende Spender im geöffneten Zustand befindet.

Die verwendeten Schmalrollen können nahezu beliebige Geometrie aufweisen. Gute Ergebnisse lassen sich mit Rollenbreiten zwischen 5 und 40 mm erzielen. Die Länge des aufgewikkelten Materials sollte mindestens etwa 20 m, bevorzugt über 500 m, pro Rolle betragen, um eine häufige Unterbrechung des Betriebes zu vermeiden. Eine Vorab-Vereinigung von zwei oder mehr Bändern und gleichzeitiges Aufwickeln bzw. Abrollen ist möglich, führt aber häufig zu Schwierigkeiten, wenn viele Schichten gewickelt werden. Es hat sich überraschend als nicht möglich erwiesen, ein aus acht oder mehr solcher Folienschichten entstandenes Laminat in einem gesteuerten Prozeß wieder abzuwickeln, da die auf dem Außenradius liegenden Laminatelemente der Folienstreifen geringfügig länger sind als die inneren Laminatelemente. Beim Abwickeln würde dies z.B. bei mehr als vier Laminatschichten zum Auseinanderlaufen der Bahnen und damit zur Prozessunterbrechung führen.
Aus diesem Grunde wird bevorzugt vom Auf- und Abrollen von Rollen mit wenigen - z.B. bis zu vier - Laminatelementen Gebrauch gemacht.

Die Art des Spenders ist dabei für die Anwendung der vorliegenden Erfindung unerheblich. Spender aus Kunststoff oder Pappe lassen sich problemlos füllen.
Die Öffnungsseite kann sich im Falle rechteckiger Folienabschnitte an der Schmalseite befinden, in welchem Fall die vorgeschnittenen Schmalrollen die Breite der geringen Seitenlänge des Rechtecks aufweisen.
Sofern die Schmalrollen aber in der späteren Längsdimension der Folienabschnitte ausgeführt sind, befindet sich die Öffnungsseite des Spenders auf einer seiner Längsseiten.

Im Falle zweiteiliger Spender würde sinnvollerweise ein z.B. schubladenförmig ausgeführtes Unterteil primär gefüllt und anschließend das Oberteil aufgesetzt werden. Es ist aber auch möglich, halbgeöffnete Spender zu befüllen.
Auch das Befüllen von Siegelbeuteln, die insbesondere im Fall einer "peel"-fähigen Ausführungsform als Spender eingesetzt werden können, ist möglich, indem z.B. der geschnittene Stapel auf die Unterbahn einer zulaufenden siegelfähigen Packstoffbahn aufgelegt und anschließend nach Zufuhr der Packstoffoberbahn der entstandene Beutel zugesiegelt wird.

Einzelheiten, Merkmale und Vorteile der Erfindung ergeben sich beispielhaft aus der nachstehenden Erläuterung eines in der Zeichnung schematisch dargestellten Verfahrensstammbaumes. Dieser zeigt:

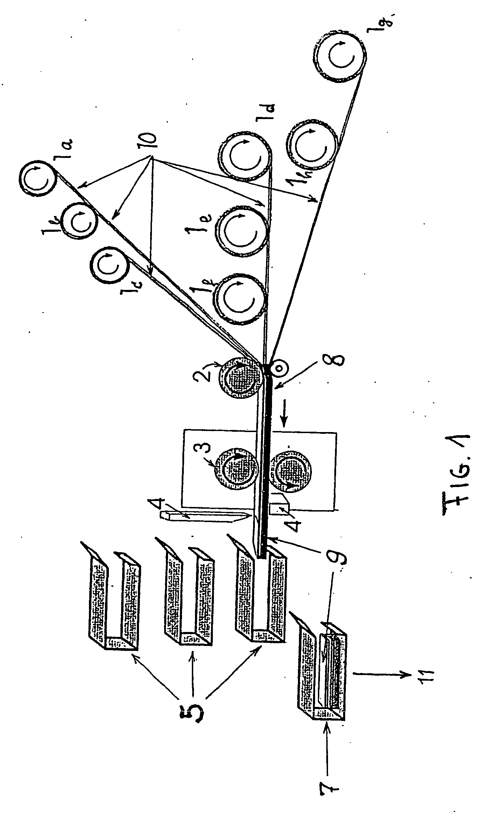

Die Vorrichtung zum Einbringen einer Vielzahl vereinzelter folienförmiger Darreichungsformen in einen Spender unter Bildung eines viellagigen Stapels (9) zur einzeldosierbaren Entnahme umfaßt gemäß Figur 1 eine unbestimmte Anzahl - je nach der Zahl der Lagen - von Rollen (1a bis 1h) mit darauf aufgewickelten wirkstoffhaltigen Bändern (10).
Diese laufen beim Abwickelvorgang mit unterschiedlichen Winkeln zur Horizontalen zu Führungsrollen (2) und zwischen diesen hindurch, wobei die Bänder (10) zu einem viellagigen Strang (8) gebündelt werden. Der Strang (8) wird von einem Paar Förderrollen (3) ergriffen und zu einer Schere (4) gezogen. Am Ende der Transportstrecke angekommen, wird mit der Schere (4) vom Strang (8) jeweils ein viellagiger Stapel (9), bestehend aus lose in exakter Ausrichtung aufeinander liegenden Darreichungsformen, abgetrennt und in einen bereitgestellten Spenderbehälter (5) eingefüllt, nachdem der Spender (5) vorher selbsttätig mit einer (nicht gezeigten) Fördereinrichtung in die Füllposition gebracht wurde. Dabei wird der Spender (5) mit wenigstens einem Stapel (9) beladen. Je nach Material und Aufgabenstellung kann ein Spenderbehälter (5) auch mit mehreren Stapeln (9) sukzessive befüllt werden.
Der beladene Behälter wird dann mit der Fördereinrichtung (11) abtransportiert.

Das Verfahren ist überraschend einfach, kostengünstig erstellbar und löst in idealer Weise die eingangs gestellte Aufgabe.

## Patentansprüche

1. Verfahren zur Herstellung eines Stapels, bestehend aus lose in exakter Ausrichtung aufeinander liegender Darreichungsformen für therapeutische, kosmetische oder lebensmitteltechhische Produkte, **dadurch gekennzeichnet, daß** durch Rollenschnitt aus ursprünglicher Breitware hergestellte Einzelbahnen aufeinandergelegt und gemeinsam quergeschnitten werden, wodurch der aus mehreren Folienschichten bestehende Mehrfachlaminatstrang unter Bildung eines Stapels folienförmiger Darreichungsformen exakt abgelängt wird.

2. Verfahren nach Anspruch 1, **dadurch gekennzeichnet, daß** die Folienschichten eine Breite zwischen 5 und 40 mm besitzen.

3. Verfahren nach Anspruch 1 oder 2, **dadurch gekennzeichnet, daß** die Folienschichten ein Flächengewicht zwischen 10 und 50 g/m² besitzen.

4. Verfahren nach einem der Ansprüche 1 bis 3, **dadurch gekennzeichnet, daß** ein Spender oder Siegelbeutel mit dem Stapel folienförmiger Darreichungsformen befüllt wird.

## Claims

1. Process for the manufacture of a stack of administration forms, lying on top of each other in exact alignment, for therapeutic, cosmetic or food-technological products, **characterised in that** individual webs, produced by roll-cutting from the original broad width material, are placed on top of each other and are jointly cross-cut, whereby the multiple-laminate strand, consisting of several film layers, is cut to a precise length, thus forming a stack of sheet-like administration forms.

2. Process according to claim 1, **characterised in that** the film layers have a width between 5 and 40 mm.

3. Process according to claim 1 or 2, **characterized in that** the film layers have a weight per unit area between 10 and 50 g/m².

4. Process according to any one of claims 1 to 3, **characterized in that** a dispenser or sealed bag is filled with the stack of sheet-like administration forms.

## Revendications

1. Procédé de fabrication d'une pile composée de formes posologiques posées librement les unes sur les autres selon un alignement précis pour des produits thérapeutiques, cosmétiques ou alimentaires, **caractérisé en ce que** des bandes continues individuelles réalisées par découpage à la molette à partir d'un produit de base large sont posées les unes sur les autres et coupées ensemble transversalement, permettant ainsi de couper la bande multistratifiée composée de plusieurs couches de feuilles à la longueur exacte en formant une pile de formes posologiques en forme de feuilles.

2. Procédé selon la revendication 1, **caractérisé en ce que** les couches de feuilles possèdent une largeur comprise ente 5 et 40 mm.

3. Procédé selon la revendication 1 ou 2, **caractérisé en ce que** les couches de feuilles possèdent un grammage compris entre 10 et 50 g/m².

4. Procédé selon l'une des revendications 1 à 3, **caractérisé en ce qu'**un distributeur ou sachet scellé est rempli de la pile de formes posologiques en forme de feuilles.
